Europäisches Patentamt

European Patent Office

Office européen des brevets

(19)

(11) Publication number: **0 101 039**

**A2**

(12) **EUROPEAN PATENT APPLICATION**

(21) Application number: **83107814.2**

(22) Date of filing: **08.08.83**

(51) Int. Cl.³: **A 61 K 39/395**
**G 01 N 33/54**

(30) Priority: **10.08.82 JP 138848/82**

(43) Date of publication of application:
**22.02.84 Bulletin 84/8**

(84) Designated Contracting States:
**CH DE FR GB IT LI**

(71) Applicant: **MEIJI SEIKA KABUSHIKI KAISHA**
**4-16 Kyobashi 2-chome**
**Chuo-ku, Tokyo-To(JP)**

(72) Inventor: **Yoshida, Kenji**
**4-30-10, Hairando**
**Yokosuka-Shi Kanagawa-Ken(JP)**

(72) Inventor: **Kimura, Fumio**
**2-1-6-401, Konandai Konan-ku**
**Yokahama-Shi Kanagawa-Ken(JP)**

(72) Inventor: **Yamada, Yujiro**
**385-13, Shimoda-Cho Kohoku-Ku**
**Yokahama-Shi Kanagawa-Ken(JP)**

(72) Inventor: **Kawaharajo, Katsumi**
**2224-33, Kamigo-Cho Totsuka-Ku**
**Yokahama-Shi Kanagawa-Ken(JP)**

(72) Inventor: **Takeda, Ueto**
**1-31-1-109, Omori Higashi**
**Ota-Ku Tokyo-To(JP)**

(74) Representative: **Dr. Elisabeth Jung Dr. Jürgen**
**Schirdewahn Dipl.-Ing. Claus Gernhardt**
**P.O. Box 40 14 68 Clemensstrasse 30**
**D-8000 München 40(DE)**

(54) Monoclonal antibody, method of producing the same and use thereof.

(57) A monoclonal antibody against *Pseudomonas aeruginosa* which is produced by a hybridoma of a cell producing an antibody against *Pseudomonas aeruginosa* and a myeloma cell and reacts specifically with *Pseudomonas aeruginosa*; a process for producing the antibody, comprising cell fusion; a reagent for diagnosis of *Pseudomonas aeruginosa* infectious disease or classification of *Pseudomonas aeruginosa*, comprising the antibody; and a therapeutical agent for *Pseudomonas aeruginosa* infectious disease, comprising the antibody are provided.

EP 0 101 039 A2

## MONOCLONAL ANTIBODY, METHOD

## OF PRODUCING THE SAME AND USE THEREOF

## BACKGROUND OF THE INVENTION

### Field of the Invention

This invention relates to a monoclonal antibody against Pseudomonas aeruginosa, a method of producing the same, and use thereof and more specifically use as a diagnostic agent for Pseudomonas aeruginosa infections or a reagent for classification of Pseudomonas aeruginosa and as a therapeutic agent for Pseudomonas aeruginosa infections.

### Description of the prior art

Pseudomonas aeruginosa has been known to be a microorganism which itself has low pathogenicity. Recently, however, as a result of alternating proliferation of microorganisms caused by administration of antibiotics, so-called opportunistic infections are increasing. Also, since this bacterium is in itself of weak toxicity, most of the patients infected with this bacterium are frequently those afflicted with, for example, cancers or a disease for which therapy with an immunosuppressant is required, patients suffering thermal burns or newborns.

Pseudomonas aeruginosa infections disease is deemed at present to be one of the infecticus diseases the therapy of which is the most difficult. Therapy of this infectious disease is difficult, partly because Pseudomonas aeruginosa is becoming resistant to various antibiotics which have heretofore been used, and also because Pseudomonas aeruginosa is liable to be resistant to pharmaceuticals recently developed which can exhibit effects to some extent against Pseudomonas aeruginosa.

When a patient infected with some bacteria, it is very important for subsequent therapy of the infectious disease to know at an early stage the identity of the bacteria. This kind of thinking has prevailed from the past, and diagnoses with a large number of anti-sera have been practiced. However, anti-sera of the prior art, as a consequence of the preparation means therefor, are, of course; mixtures of antibodies with different specificities produced by a great number of clones, namely, polyclonal antibodies. Therefore, it has been difficult to produce antibodies of a constant quality, and a number of operations are necessary for suppressing non-specific reactions. These and many other problems have been pointed out.

## SUMMARY OF THE INVENTION

1. Gist

An object of the present invention is to provide a solution to the above problems, and it is intended to accomplish this object by producing a monoclonal antibody against Pseudomonas aeruginosa and using the same.

Accordingly, the monoclonal antibody against Pseudomonas aeruginosa according to the present invention is characterized in that it is produced by a hybridoma of a cell capable of producing an antibody against _Pseudomonas aeruginosa_ and a myeloma cell and reacts specifically with _Pseudomonas aeruginosa_.

The antibody against _Pseudomonas aeruginosa_ is produced according to the present invention by a process which comprises subjecting a cell capable of producing an antibody against _Pseudomonas aeruginosa_ and a myeloma cell to cell fusion to form a hybridoma between both cells, proliferating the hybridoma and obtaining antibodies against _Pseudomonas aeruginosa_ produced by the hybridoma.

The present invention also concerns use of the antibodies.

The reagent for diagnosia of _Pseudomonas aeruginosa_ infectious diseases or for classification of _Pseudomonas aeruginosa_ according to the present invention comprises at least one kind of monoclonal antibody against _Pseudomonas aeruginosa_.

The therapeutical agent for _Pseudomonas aeruginosa_ infectious diseases according to the present invention comprises at least one kind of monoclonal antibody against _Pseudomonas aeruginosa_.

2. Advantageous Effect

By the use of the monoclonal antibody of the present invention, various problems recognized in the aforesaid polyclonal antibody can be overcome.

What is of interest in the present invention is that the monoclonal antibody is obtained as globulin, primarily of the IgM type. As a result, the antigen-antibody reaction with the bacteria can be detected within a very short time without accompaniment of the problem, as observed in the monoclonal antibody of the prior art, of the impossibility of using a method such as agglutination reaction or sedimentation reaction with a polyclonal antibody. The antibody of the present invention can also be obtained as one of IgG type, which can be adsorbed onto resin particles of a resin emulsion which can undergo rapid agglutination.

DETAILED DESCRIPTION OF THE INVENTION

1. Pseudomonas aeruginosa employed

There is much controversy over classification and identification of Pseudomonas aeruginosa even at the present time, and much difference and confusion exist in classifications of Pseudomonas aeruginosa according to type of sera even among the researchers. This situation may be considered to be caused greatly by the facts that, due to the use of polyclonal antibodies, the subjects of researches have not been integrated or made common on account of the difference between individual animals used, the difference in the conditions for absorption operations of anti-sera used, etc.

In the present invention, the classification of Pseudomonas aeruginosa to be used follows the serological

classification according to the determination in 1975 by The Serotyping Committee for the Japan <u>Pseudomonas aeruginosa</u> Society, and microorganism strains belonging to the groups A - M according to this classification are employed herein.

Some of the microorganism strains of <u>Pseudomonas aeruginosa</u> belonging to the groups A - M are stored at the Institute for Research of Medical Science of the University of Tokyo (Bunkyo-ku, Tokyo) and are freely releasable to a third party.

The strains of <u>Pseudomonas aeruginosa</u> stored at the Institute for Research of Medical Science of the University of Tokyo are shown below. For the purpose of reference, classification by Honma et al are also shown in the same Table.

6

Table 1

| Strain | Classification by Society for Study of Pseudomonas aeruginosa | Classification by Honma et al |
|--------|---|---|
| IID 1001 | A | 1 |
| IID 1002 | B | 2 |
| IID 1007 | " | 7 |
| IID 1013 | " | 13 |
| IID 5004 | " | 16 |
| IID 1021 | C | 3 |
| IID 1004 | D | 4 |
| IID 1130 | E | 5 |
| IID 1006 | F | 6 |
| IID 1020 | G | 8 |
| IID 1009 | H | 9 |
| IID 1010 | I | 10 |
| IID 1011 | J | 11 |
| IID 1012 | K | 12 |
| IID 5141 | L | 14 |
| IID 5018 | M | 15 |
| IID 1015 | " | 17 |

In the present invention, the serological classification according to the determination by The Serotyping Committee has been judged to be the best and is followed as the classification of Pseudomonas aeruginosa. However, since the classification of the groups A - M is an index for the time being, it is possible that a new classification standard will be proposed or adopted in the future. In particular, it is not difficult to imagine that, with the progress of classification and identification by use of the monoclonal antibody according to the present invention, there may be formed microorganism strains for which the present classification standard will not be applicable. Thus, the present invention is not limited, with respect to the microorganism strains to be used, to only those belonging to the above groups A - M or those which can be classified according to this classification standard.

2.  Preparation of monoclonal antibody

The monoclonal antibody against Pseudomonas aeruginosa of the present invention is produced according to the cell fusion method.

Production of a monoclonal antibody according to the cell fusion method comprises, generally, (1) preparation of cells producing antibodies for cell fusion, (2) cell fusion/preparation of hybridoma, (3) screening and cloning (and storage) of a desired hybridoma, and (4) production of the desired monoclonal antibody by proliferation of the hybridoma.

These unit steps, and therefore the method for preparation of a monoclonal antibody comprising a combination thereof, are known, and the known method can be utilized also in the present invention, with the proviso that there may possibly be modifications based on the microorganism used as the antigen, which is Pseudomonas aeruginosa.

As a general description of preparation of hybridoma and production of a monoclonal antibody, reference may be made to J. Immunol. Methods 39, 285 - 308 (1980).

An example of the method of producing a monoclonal antibody according to the present invention will now be described in detail.

1) Preparation of a cell producing antibody for cell fusion:

Microorganism strains belonging to the groups A - M of Pseudomonas aeruginosa are each cultured and, preferably after treatment to have them weakly toxic or non-toxic by e.g. formalin treatment, the washed microorganism cells or lipopolysaccharides of microorganism cells or the membrane components of microorganism cells are administered intraperitoneally or subcutaneously (e.g., at the back, or the joints of limbs) into host animals such as mice, particularly BALB/C mice.

About two weeks after administration, the same microorganism is administered again for additional immunization or additional sensitization, after which, if necessary, additional immunization is carried out. Additional immunization is conducted through intravenous injection in many cases.

Finally, 72 hours after immunization, spleens are extirpated to provide antibody-producing cells (lymphocytes).

2)  <u>Cell fusion/Preparation of hybridoma</u>:

Myeloma cells previously cultured, such as those of P3-X63-Ag8 [Cold Spring Harbor Symp. Quant. Biol., 41, 781-791 (1976)], X63-Ag8-6.5.3 and others, are mixed with the above described spleen cells in a ratio of about 1:1 to 1:10, and fusion of both cells is caused according to the known method [J. Immunol. Methods <u>39</u>, 285-308 (1980)] with addition of an appropriate medium for cell fusion such as RPMI-1641 medium (e.g., Nissui) containing about 40% polyethylene glycol (PEG) (MW: ca. 1,000 - 6,000) and about 15% dimethyl sulfoxide (DMSO) to form hybridoma, which step is followed by exchanging of the medium with that suitable for growth of the hybridoma alone, to obtain a cultured product of hybridoma alone.  As the medium for growth of hybridoma alone, when the myeloma cells employed are the specific examples as mentioned above, HAT medium (RPMI-1640 medium containing hypoxanthine, aminopterin and thymidine) is generally employed in view of their metabolic or biosynthetic characteristic (lymphocytes of spleen cannot be proliferated for a long term under the <u>in vitro</u> conditions as in HAT medium).

3)  <u>Screening and cloning of a desired hybridoma</u>:

One week to two weeks after growth of the hybridoma in HAT medium as described above, the aforesaid <u>Pseudomonas</u>

aeruginosa microorganism cells are allowed to react with the hybridoma supernatant in a micro-plate. After washing by centrifugation of the reacted Pseudomonas aeruginosa microorganism, screening of the hybridoma producing the antibodies reactive specifically with the objective bacteria is conducted according to the enzyme immunoassay (EIA) using anti-mouse antibodies labelled with a peroxidase.

For the hybridoma, the desired activity of which has been detected by screening, cloning is performed by, for example, single-cell-manipulation under microscopic observation. After about two weeks, for the hybridoma of which only one clone is grown per well, EIA is carried out again to establish the clone.

The established clone can be stored by a measure such as frozen state in liquid nitrogen, except for one to be immediately used for monoclonal antibody production.

4)  Production of monoclonal antibody:

The hybridoma of the established clone can be proliferated by in vitro cultivation in an appropriate medium or by in vivo cultivation in mouse peritonea to produce a desired monoclonal antibody.

For example, the established clone is subjected gradually to scale-up according to conventional procedures (desired antibodies are contained in the cultural supernatant), and in order to obtain a specimen of still higher antibody value, a hybridoma controlled to a number of cells of $1 - 10 \times 10^6/0.5$ ml is administered intraperitoneally into a BALB/C mouse previously administered one to two weeks before intraperitoneally with pristane (Aldrich). About one to two weeks after administration, the desired monoclonal antibody with an antibody value about 10 to 500 times that of the hybridoma cultural supernatant is contained in the ascite and the serum deposited.

The desired antibody in the culture broth or the ascite thus obtained may be utilized as it is, but it is preferably purified to a high antibody value specimen.

For the purification, any method employed for purification of immunoglobulins can be employed. The salting-out method with ammonium sulfate, the ionexchange method with the use of DEAE cellulose, etc., the gel filtration method, affinity-chromatography, etc., are suitable. The desired antibody with high purity can be obtained by a suitable combination of these methods or a combination thereof with electrophoresis.

The phrase "to obtain antibodies" in the production method according to the present invention is also inclusive of the case of obtaining antibodies in the form of a culture broth or a ascite in addition to the case in which antibodies are obtained as purified preparation.

3.  Use of monoclonal antibody

The monoclonal antibody thus obtained can be provided for various uses by utilizing its specificity against Pseudomonas aeruginosa.

Some examples of application are shown below.

1)  Reagent for diagnosis of Pseudomonas aeruginosa infectious diseases or for classification of the microorganism:

The monoclonal antibody according to the present invention can clearly distinguish between the Pseudomonas aeruginosa of the A - M groups even in the form of a hybridoma cultural supernatant, a ascite and a serum, other than purified preparation.  That is, as confirmed by the present inventor, the monoclonal antibody produced by the hybridoma obtained by sensitization with the Pseudomonas aeruginosa belonging to the group A reacts only with the microorganism of the group A and does not react at all in EIA with any of the other groups B - M. Similarly, the antibody from the microorganism of the group M reacts only with the microorganism of the group M, as clearly distinguished from those of the groups A - L.

The monoclonal antibodies according to the present invention are obtained against respective Pseudomonas aeruginosa of the groups A - M, and distinction between the groups of A - M may be possible as described above. However, it is possible, as stated hereinbefore, that some Pseudomonas aeruginosa belonging to other groups than the groups A - M will be found in the future, or further sub-groups will be found for any of the A - M groups, or an entirely different classification will be adopted. Therefore, the reagent for diagnosis or classification is not limited to the existing A - M groups.

In diagnoses of infectious diseases, only identification of the microorganism as _Pseudomonas aeruginosa_ is sometimes needed, no further knowledge about its attribution to any group being required. In such a case, it is not expedient to make judgement one by one for the groups A - M. In such a case, it is reasonable to employ a mixture comprising a combination of all kinds or some kinds of the monoclonal antibodies (herein called unit anbitodies) for the respective groups of A - M.

The term "_Pseudomonas aeruginosa_ classification" herein used is inclusive also of the cases in which classifications of _Pseudomonas aeruginosa_ are generally practiced, in addition to the case in which the microorganism is classified in infectious diseases.

For diagnosis, simplicity is required simultaneously with precision. The diagnostic agent containing plural kinds of the above unit antibodies satisfies this requirement with respect to the point that the number of diagnoses can be reduced. Further, the reagent for classification of the present invention also satisfies this requirement with respect to the point of

14

easy classifying work, since some of the monoclonal antibodies of the present invention can cause agglutination of Pseudomonas aeruginosa within a very short period of time, while some can be made into appropriate "preparation forms" to make possible a short time agglutination.

That is, generally speaking, monoclonal antibodies which have very high specificities are believed to cause no agglutination reaction or sedimentation reaction in agar as observed in polyclonal antibodies during the objective specific antigen-antibody reactions. This is the reason for the advantage of precise diagnosis or classification by use of a monoclonal antibody. On the other hand, this advantage has led to a problem in that occurrence of the specific antigen-antibody reaction cannot be easily determined because of the absence of agglutination reaction or sedimentation reaction in some monoclonal antibodies. Most of the monoclonal antibodies according to the present invention are immunoglobulins belonging to the IgM type, and these antibodies were found by the present inventor to cause specific agglutination reaction within a very short time when mixed with the corresponding Pseudomonas aeruginosa. Therefore, the specific antigen-antibody reaction can be detected very easily. This is entirely unexpected from the above described knowledge concerning monoclonal antibodies.

Some of the monoclonal antibodies are obtained as immunoglobulins of IgG type. In the case of a monoclonal antibody of IgG type, no agglutination occurs by itself, as is generally known in the art. However, even such antibodies, when adsorbed onto a suitable carrier such as that of resin particles of a resin latex, can cause specific agglutination reactions of microorganism within a very short period of time, similarly as in the case of antibodies comprising IgM. Therefore, also in case of antibodies comprising IgG, specific antigen-antibody reactions can be detected very easily, whereby the requirement of simplicity in diagnosis or classification is met. When there is no restriction with respect to time in diagnosis or classification, it is possible to make precise judgement by the use of EIA, etc. for either of the antibodies comprising IgG and IgM.

For preparation of a diagnostic agent or a classification agent, any measure suited for the purpose of use to obtain such an agent in any desired preparation form can be employed. For example, a ascite, a cultured broth containing a desired antibody, or a purified antibody is subjected to assay of its antibody value and diluted suitably with PBS (phosphate buffer containing isotonic sodium chloride), for example, and then 0.1% sodium azide, for example, is added thereto as a preservative. Also, the antibodies adsorbed on a carrier such as latex may be suitably diluted after determination of the antibody value thereof and used with addition thereto of a preservative.

As mentioned above, the antibodies of the present invention adsorbed onto latex particles are one of the preferable "preparation forms" of the diagnostic agent or classification agent of the present invention. As the latex in this case, latexes of polystyrene, polyvinyl toluene, polybutadiene, etc., are suitable. [Reference is made to American Journal of Medicine 21, 888-892 (1956), concerning latex carriers]. In this connection, a polystyrene latex having human γ-globulin adsorbed thereon is being practically used as a reagent for detection of rheumatoid factors.

2) Therapeutical agent for Pseudomonas aeruginosa infectious diseases:

When experiments relating to therapy of Pseudomonas aeruginosa infectious diseases in mice were conducted by using the monoclonal antibody according to the present invention, a small amount of the antibody was found by the present inventor to have a very potent therapeutical effect specifically on the corresponding group of the microorganism. This experimental result indicates the possibility of utilization of the monoclonal antibody of the present invention as a therapeutical agent for animals (including humans) having Pseudomonas aeruginosa infectious diseases.

4. <u>Experimental examples</u>

<u>Example 1</u>. Production of a monoclonal antibody

1) Experimental method

(1) Microorganism strains employed:

Microorganism strains belonging to the groups A - M based on the serological classification as determined by The Serotyping Committee sponsored by the Society for Study of <u>Pseudomonas</u> <u>aeruginosa</u>, Japan were procured from the Institute for Research of Medical Science of the University of Tokyo and used.

(2) Cultivation of microorganism:

Each microorganism strain previously cultured on an agar slant medium at 37°C overnight was scraped off with a platinum loop into Sakaguchi's flask containing 100 ml BHI medium (Difco), or 1 to 2 ml of a broth from the seed-culture in 10 ml of BHI medium was inoculated into Sakaguchi's flask containing 100 ml of BHI medium, which step was followed by shaking of the cultivation at 37°C for 17 to 24 hours.

(3) Treatment of microorganism cells:

The culture broth (100 ml) cultivated according to the above method was subjected to centrifugation at 10,000 rpm for 20 minutes. The microorganism cells obtained were thoroughly mixed with 10 ml of 10% formalin, and the mixture was left to stand at 37°C overnight and stored in a refrigerator before use. The formalin-treated microorganism cells were thoroughly washed with a phosphate buffer containing isotonic sodium chloride (pH 7.2, abbreviated hereinafter as PBS) for removal of formalin before use, controlled to a desired concentration with PBS based on the value at $OD_{550 \, nm}$, and provided for use in sensitization of mice and analysis of antibodies.

(4)  Preparation of lipopolisaccarides (hereinafter abbreviated as LPS) of respective microorganism cells:

The microorganism cells cultured according to the foregoing method were collected by centrifugation, washed with 0.12 M Tris buffer (pH 8), suspended in 50 ml of Tris, adjusted to 37°C, and then stirred with addition of 50 ml of Tris containing 0.02 M EDTA.  Four minutes later, $MgCl_2$ was added to a final concentration of 0.05 M, and the mixture was subjected to centrifugation at 10,000 rpm for 30 minutes.  The supernatant was collected.  The resultant supernatant was dialyzed against a 0.1-M phosphate buffer and then against purified water and was thereafter subjected to lyophilization to obtain LPS of respective microorganisms.

(5)  Sensitization of mice with antigens:

In the case of sensitization with microorganism cells, the formalin-treated cells were thoroughly washed with PBS, then suspended in PBS, adjusted to 0.1 to 0.2 of OD value at 550 nm, and thereafter administered in an amount of 0.2 to 0.3 ml intraperitoneally into BALB/C mice of ages of 4 to 8 weeks. On the other hand, in the case of LPS, the lyophilized LPS was dissolved in PBS, adjusted to a final concentration of 5 to 20 µg/0.2 ml, and administered in an amount of 0.2 ml intraperitoneally.

(6) Hybridization:

At least two weeks after sensitization of the animals with the microorganism cells or LPS, the same antigens were administered for additional sensitization through the tail veins in an amount of 0.5 ml of a suspension with OD value at 550 nm of 0.2 to 0.5 in the case of microorganism cells, and of 20 to 30 µg in the case of LPS. The mice were anesthesized with chloroform or ethyl ether 72 hours after additional sensitization, and their spleens were aseptically extirpated. The spleens obtained were washed with RPMI-1640 medium (Nissui) and cut into pieces with scissors to obtain sample spleen cells. The spleen cells obtained were washed with RPMI-1640 medium containing no fetal calf serum (hereinafter abbreviated as FCS).

On the other hand, the myeloma cells (P3-X63-Ag8-Ul cells) at the logarithmic proliferation stage after previous cultivation in RPMI-1640 medium containing 10 µg/ml of 8-azaguanine and 10% FCS at 5% $CO_2$/relative humidity of 100%/37°C were washed with RPMI-1640 medium containing no FCS, and then mixed with the previously described spleen cells in a ratio in number of cells of 10:1 to about 1:1. The mixed cells were centrifuged for 10 to 15 minutes, the supernatant being discarded, and the cells were thoroughly loosened into individual cells.

Into the centrifugal tube containing the cells was gently added 0.5 ml of a solution consisting of 2 g of a polyethylene glycol (M.W. = 100 - 6000, produced by Nakarai Kagaku, Japan), 2 ml of RPMI-1640 medium (containing no FCS) and 0.7 ml of DMSO (Wako Junyaku, Japan), after which mixing was carried out while the centrifugal tube was slowly rotated. One minute later, while 1 ml of a medium containing no FCS was slowly added, the centrifugal tube was gently rotated to carry out mixing. Then, 1 ml of the medium was added every 30 seconds, and the same manners were repeated until 10 ml of the medium had been added. Upon completion of the addition of 10 ml of the medium, the mixture was subjected to centrifugation at 1,000 rpm for 10 to 15 minutes, the supernatant being discarded, and the cells were thoroughly loosened into individual cells. Then, with addition of the RPMI-1640 medium containing 10% FCS to $1 \times 10^5 - 1 \times 10^3$ cells/0.1 ml as myeloma cells, the mixture was apportioned each 0.1 ml/well into a plastic 96-well microplate (Nung), which step was followed by cultivation at 37°C/5% $CO_2$/relative humidity of 100%. The next day, 0.1 ml of a RPMI-1640 medium containing 0.387 mg/100 ml of hypoxanthine, 0.0176 mg/100 ml of aminopterin, 1.3 mg/100 ml of thymidine (each being produced by Sigma) and 10% FCS (hereinafter abbreviated as HAT medium) was added, and 1/2 of the medium was replaced on the second day and the third day after fusion with HAT medium. Then, every 3 to 4 days, the medium was gently removed with addition of HAT medium to await the growth of the hybridoma. About one to two weeks later, growth of the hybridoma could be observed in the well under a microscope.

(7) Screening of desired antibodies:

RPMI-1640 medium containing 5% rabbit serum was added to a 96-well polyvinyl chloride plate (Nung) and left to stand at 4°C overnight. Then, the medium was discarded, and 100 μl of the sample to be assayed and 50 μl of the microorganism suspension ($OD_{550} \approx 10$) for the antigen were added and incubated at 37°C for 60 minutes. After centrifugation at 2,000 rpm for 20 minutes and washing with the medium repeated twice, 50 μl of 100-fold dilution of anti-mouse antibodies (Cappel) labelled with a peroxidase was added, and the reaction was carried out at 37°C for 60 minutes. After the reaction, the product was washed three times with PBS, and 100 μl of 0.1% o-phenylenediamine solution (0.1 M citrate buffer containing 2 μl of 30% $H_2O_2$ in 10 ml, pH 4.5) was added thereto. The mixture was left to stand at room temperature. After 30 minutes, antibodies of yellowish brown color were judged with naked eye and selected.

(8)  Cloning of cells producing desired antibodies:

The two methods described below were employed.

(a)  Thymus cells or spleen cells of a BALB/C mouse are suspended in HAT medium to $1 \times 10^6/0.2$ ml, and the suspension is previously apportioned in a proportion of 0.2 ml/well.  The hybridoma in the well which has been judged to have produced the desired antibody as the result of screening is peeled off and mixed thoroughly by means of a pipette, and then a part thereof is sampled in a dish.  Then HAT medium is added to prepare a thin suspension of the cells.  Under microscopic observation, the cells are picked up one by one with a glass capillary and placed into the wells, one to each well (one cell/well), in the 96-well micro-plate containing thymus or spleen cells which has been previously prepared.

(b).  A suspension of the hybridoma in the well judged to have formed the desired antibody as the result of screening is prepared and, after examination of the cell number accurately by the use of a blood cell counting plate, is diluted with HAT medium and finally added to a medium containing thymus or spleen cells, thereby to prepare a suspension of cells containing 0.3 to 0.5 cells/0.2 ml as the hybridoma and $1 \times 10^6$ cells/ 0.2 ml of thymus or spleen cells.  This suspension is apportioned into portions each of 0.2 ml into the 96-well microplate.

When the microplate after cloning according to the method (a) or (b) as described above is cultured at 5% $CO_2$/37°C, clones are grown after one to two weeks. The wells which have produced only one clone per well are chosen, and each clone is analyzed according to the method described in the paragraph on screening to choose the clones which have produced the desired antibody.

(9)   In vitro cultivation of cells and production of antibodies:

The desired clones which had been amply proliferated in the 96-well microplate were cultured in gradually scaled up plates of 24 wells, 6 wells and 4 wells, and further on a greater scale according to necessity. The supernatant of the culture of the cells thus obtained contained antibodies produced therein which reacted specifically with the target microorganism cells.

2)   Results

The microorganism strains used in sensitization, immunization and reaction were as shown above in Table 1.

Table 2 shows the relationships between the monoclonal antibodies produced by the representative hybridomas obtained by the present inventors and the reactivities (by the EIA method) with the microorganisms of the respective groups.

The monoclonal antibodies specific for respective groups obtained by the present inventors were examined to determine the class of the antibodies as immunoglobulins.

As a result of the investigation on IgM which is instable to SH reagents such as mercaptoethanol, dithiothreitol, etc. and IgG which is stable thereto [Pirofsky et al: Vox sang. 27: 480-488 (1974)] and also on the reactivities with commercially available μ-chain specific and γ-chain specific anti-mouse immunoglobulins and agglutination tendencies of the monoclonal antibodies relative to the corresponding groups of microorganisms, the antibodies obtained were found to belong to IgM or IgG. Table 3 shows one example of the results.

Example 2  Experimental infection therapy

One example of an experiment in which a hydroperitoneum containing monoclonal antibody is used will now be described.

One day before and 4 hours before infection of ICR-strain mice (male, 4 weeks old) with Pseudomonas aeruginosa, 0.2 ml of a ascite containing a monoclonal antibody or a dilution thereof with isotonic sodium chloride solution was ip administered. $LD_{50}$ values of the respective Pseudomonas aeruginosa for the mice were previously examined and 10- to 20-fold amounts of $LD_{50}$ of microorganisms were suspended in 25% mucin and ip administered to the mice. Observation was continued one week thereafter for examination of the survival number. As a Control, a ascite obtained by administering myeloma to mouse was used without dilution. The results are shown in Table 4.

All mice were dead on the next day, but strong therapeutical effects were observed in groups which had been administered with respective corresponding antibodies. The therapeutical effects were group-specific, and the effect against the microorganism of the group A was possessed only by the group A-specific antibodies, no effect being observed in the antibodies with different types.

Table 2

| Group of microorganism | A | B | | | | C | D | E | F | G | H | I | J | K | L | M | |
|---|---|---|---|---|---|---|---|---|---|---|---|---|---|---|---|---|---|
| Monoclonal antibody No. | 1 | 2 | 7 | 13 | 16 | 3 | 4 | 5 | 6 | 8 | 9 | 10 | 11 | 12 | 14 | 15 | 17 |
| 1 | + | | | | | | | | | | | | | | | | |
| 2 | | + | | | | | | | | | | | | | | | |
| 3 | | | + | | | | | | | | | | | | | | |
| 4 | | | | + | | | | | | | | | | | | | |
| 5 | | | | | + | | | | | | | | | | | | |
| 6 | | | | | | + | | | | | | | | | | | |
| 7 | | | | | | | + | | | | | | | | | | |
| 8 | | | | | | | | + | | | | | | | | | |
| 9 | | | | | | | | | + | | | | | | | | |
| 10 | | | | | | | | | | + | | | | | | | |
| 11 | | | | | | | | | | | + | | | | | | |
| 12 | | | | | | | | | | | | + | | | | | |
| 13 | | | | | | | | | | | | | + | | | | |
| 14 | | | | | | | | | | | | | | + | | | |
| 15 | | | | | | | | | | | | | | | + | | |
| 16 | | | | | | | | | | | | | | | | + | + |
| 17 | | | | | | | | | | | | | | | | | + |
| 18 | + | | | + | | | | | | | | | | | | | |
| 19 | | | + | | + | | | | | | | | | | | | |
| 20 | | + | | + | + | | | | | | | | | | | | |
| 21 | | + | + | + | + | | | | | | | | | | | | |
| 22 | | + | + | | + | | | | | | | | | | | | |
| 23 | | + | + | | | | | | | | | | | | | | |
| 24 | | | | | | + | + | | | | | | | | | | |

+: Reactive, Vacant column: Not reactive

Table 3

| Monoclonal antibody | Group specificity | SH reagent stability | μ-chain or γ-chain | Agglomeration of microorganism | Judgement of Ig class |
|---|---|---|---|---|---|
| a | Group A | Unstable | μ | + | IgM |
| b | " | " | " | " | " |
| c | I | Stable | γ | − | IgG |
| d | " | " | " | " | " |
| e | H | " | " | " | " |
| f | " | Unstable | μ | + | IgM |
| g | L | " | " | " | " |
| h | " | Stable | γ | − | IgG |

Table 4

| Sample No. | Group of micro-organism | $LD_{50}$ microorganism amount/mouse | Amount of sensitizing microorganism/mouse | Control | Dosage (dilution) | | | |
|---|---|---|---|---|---|---|---|---|
| | | | | | x1 | x10 | x100 | x1000 |
| 1 | A | $1 \times 10^4$ | $1 \times 10^6$ | $0/6^*$ | $6/6^*$ | $6/6^*$ | $5/6^*$ | $3/6^*$ |
| 2 | F | $1 \times 10^7$ | $2 \times 10^8$ | $0/6$ | $6/6$ | $5/6$ | $5/6$ | $2/6$ |
| 3 | G | $1 \times 10^4$ | $1 \times 10^6$ | $0/6$ | $6/6$ | $6/6$ | $6/6$ | $3/6$ |
| 4 | L | $1 \times 10^6$ | $1 \times 10^7$ | $0/6$ | $6/6$ | $6/6$ | $5/6$ | $2/6$ |

* Numerator: Survival number of mice, Denominator: Number of mice tested

WHAT IS CLAIMED IS:

1.     A monoclonal antibody against <u>Pseudomonas</u> <u>aeruginosa</u> which is produced by a hybridoma of a cell producing an antibody against <u>Pseudomonas</u> <u>aeruginosa</u> and a myeloma cell and reacts specifically with <u>Pseudomonas</u> <u>aeruginosa</u>.

2.     A monoclonal antibody according to claim 1, comprising mainly globulin selected from the group consisting of IgM globulin and IgG globulin.

3.     A monoclonal antibody according to claim 2, comprising mainly IgM globulin.

4.     A reagent for diagnosis of <u>Pseudomonas</u> <u>aeruginosa</u> infectious disease or for classification of <u>Pseudomonas</u> <u>aeruginosa</u>, comprising at least one kind of monoclonal antibody against <u>Pseudomonas</u> <u>aeruginosa</u>.

5.     A reagent according to claim 3, wherein the monoclonal antibody comprises mainly globulin selected from the group consisting of IgM globulin and IgG globulin.

6.     A reagent according to claim 5, wherein the monoclonal antibody comprises mainly IgM globulin.

7.    A reagent according to any one of claims 4 to 6, wherein the monoclonal antibody is adsorbed on resin particles in a resin emulsion.

8.    A reagent according to claim 7, wherein the monoclonal antibody is IgG globulin.

9.    A therapeutical agent for a <u>Pseudomonas</u> <u>aerugi-</u><u>nosa</u> infectious disease, comprising at least one kind of monoclonal antibody against <u>Pseudomonas</u> <u>aeruginosa</u>.

10.    A process for producing a monoclonal antibody against <u>Pseudomonas</u> <u>aeruginosa</u>, which comprises subjecting a cell capable of producing an antibody against <u>Pseudomonas</u> <u>aeruginosa</u> and a myeloma cell to cell fusion to form a hybridoma between both cells, proliferating said hybridoma and obtaining antibodies against <u>Pseudo-</u><u>monas</u> <u>aeruginosa</u> produced by said hybridoma.